# EUROPÄISCHE PATENTSCHRIFT

(11) **EP 1 183 055 B1**
(45) Veröffentlichungstag und Bekanntmachung des Hinweises auf die Patenterteilung: **26.03.2003**
(21) Anmeldenummer: 00918647.9
(22) Anmeldetag: 28.04.2000
(51) Int. Cl.: A61L 9/14

(54) **PARFUMDISPENSER**
PERFUME DISPENSER
DIFFUSEUR DE PARFUM

(30) Priorität: 25.05.1999 CH 97899
(43) Veröffentlichungstag der Anmeldung: 06.03.2002
(73) Patentinhaber: Masanis Art, Food & Fashion Ltd., 8057 Zürich (CH)
(72) Erfinder: DROZ, Christian, CH-5600 Lenzburg (CH)
(74) Vertreter: Patentanwälte Feldmann & Partner AG
(86) Internationale Anmeldenummer: CH0000238
(87) Internationale Veröffentlichungsnummer: WO00071174

(56) Entgegenhaltungen:
- EP-A- 0 753 293
- DE-A- 2 122 184
- US-A- 2 844 893

## Beschreibung

Die vorliegende Erfindung betrifft einen Parfumdispenser gemäss Oberbegriff des Patentanspruch 1.

Öffentliche Nassräume wie Waschräume oder Toiletten werden von vielen Menschen nur ungern aufgesucht, können jedoch den Eindruck eines Kunden von einer Lokalität ganz entscheidend prägen.
Die Inhaber von Restaurants, Kinos, Warenhäusern etc. sind daher bestrebt den Kunden die Nassräume nicht nur hygienisch einwandfrei zu präsentieren, sondern durch weitere Massnahmen den Aufenthalt möglichst angenehm zu gestalten. So werden zum Beispiel in vielen Waschräumen oder Toiletten Raumsprays verwendet. Dazu sind meistens an einer erhöhten, unzugänglichen Stelle im Raum, zum Beispiel unterhalb der Decke Dispenser montiert. Aus einer handelsüblichen Spraydose wird zum Beispiel aktiv in regelmässigen Intervallen mittels eines elektrischen Betätigungsmechanismus oder passiv, zum Beispiel durch das Öffnen der Waschraum- oder Toilettentüre, Raumspray abgegeben.

Die Erfindung gemäss der DE 2'122'184 besteht darin, dass eine an sich optisch unschöne Spraydose in eine Verkleidung bzw. Verpackung, bestehend aus Rückwand, Boden, Seitenwänden und Vorderwand, gegeben wird, letztere als Spiegel, Kalender, Blumen- oder Kunstbild ausgebildet ist und deren Rückwand zu ihrer Befestigung an einer Wand, einer Tür oder dergl. vorbereitet ist. Es ist eine Vorrichtung zum Aufhängen der Verkleidung und damit der Spraydose an einer Wand, eine Öffnung zum Durchlass eines Schnurzugs oder einer anderen Vorrichtung zur Betätigung des Sprühventils und oben eine Öffnung für den Durchtritt des Zerstäuberstrahls vorhanden. Durch die Erfindung ist der störende mit Reklameaufdrucken und Bedienungsvorschriften versehene Sprühdosenzylinder nicht mehr zu sehen. Die Verpackung mit der Spraydose ist so an der Wand befestigt, dass der Zerstäuberstrahl festgelegt und so gerichtet ist, dass der sich zunehmend erweiternde Strahlkegel nicht an die Wand treffen kann, an welcher die Spraydose angehängt ist.

Ein Glasbehälter zur Aufnahme von Pharmazeutika, wie sie vorzugsweise von Apothekern zum Abfüllen von Medikamenten verwendet werden ist in der US-2'844'893 beschrieben. An den Behältern können Etiketten ohne den Einsatz von Klebstoff angebracht werden. Die Etiketten kommen zwischen einer inneren Behälterwend und einem transparenten Schutzmantel zu liegen.

Zur dosierten Abgabe von Flüssigseife werden ebenfalls verschiedene Arten von Dispensern eingesetzt. Üblicherweise sind diese Dispenser an der Wand in Reichweite der Waschbecken montiert. Durch mechanische Betätigung eines Hebels oder Knopfes wird eine vorgegebene Dosis Flüssigseife aus dem Spender gepumpt. Um den Verbrauch an Seife zu verringern wird bei neueren Modellen die abgegebene Seife bei der Abgabe mit Luft aufgeschäumt.
Der positive Eindruck auf die Benützer eines öffentlichen Nassraumes kann weiter gesteigert werden, indem eine Möglichkeit geboten wird sich nach Wunsch mit Parfum oder Eau de Cologne zu erfrischen.
Dazu können wiederum Dispenser verwendet werden, die an gut zugänglichen Stellen montiert sind.
Die bekannten Dispenser zur Abgabe von Flüssigseife sind hierzu jedoch unbrauchbar, da sie es nicht erlauben das Duftwasser in Form eines feinverteilten Aerosols abzugeben.
Die bekannten Dispenser für Raumsprays mit integrierter Spraydose sind für diesen Zweck ebenfalls nicht geeignet. Der Inhalt der Spraydosen kann nur dann annähernd vollständig abgegeben werden, wenn die Spraydose aufrecht steht. Das heisst der Sprühnebel tritt immer an einer Oberseite der Spraydose aus.
Es ist daher die Aufgabe der vorliegenden Erfindung, einen Parfum- oder Eau de Cologne-Dispenser zur Verfügung zu stellen, bei dem das Aerosol an einer Unterseite des Dispensers austritt. Dadurch soll einerseits ein bequemes Besprühen der Hände ermöglicht werden. Andererseits soll, aus Gründen die nachfolgend noch dargelegt werden, der Dispenser in einer Höhe montierbar sein, dass er sich im Gesichtsfeld des Benützers befindet. Der Benützer soll den Dispenser beim Betätigen voll im Blick haben, da der Dispenser gleichzeitig als Werbeträger dient. Da sich ein solcher Dispenser an öffentlich zugänglichen Orten befindet und der Inhalt einen nicht unerheblichen Wert besitzt, muss ausserdem sichergestellt sein, dass er nicht von Unbefugten entfernt werden kann. Dispenser und Inhalt sollen zudem vor Beschädigungen oder Manipulationen bestmöglich geschützt und möglichst unanfällig für Störungen sein. Nachfüll- und Service-Intervalle sollen auch für Dispenser in stark frequentierten Örtlichkeiten sehr lange gehalten werden können.

Diese Aufgabe löst ein Parfumdispenser mit den Merkmalen des Patentanspruches 1.

In den beiliegenden Zeichnungen sind Ausführungsbeispiele des Erfindungsgegenstandes dargestellt, die in der nachfolgenden Beschreibung erläutert werden. Es zeigen:
- Figur 1a: eine Ansicht von vorne auf einen erfindungsgemässen Parfumdispenser;
- Figur 1b: eine Seitenansicht eines Parfumdispenser gemäss Fig. 1a in Wandmontage;
- Figur 2a: eine Ansicht von vorne auf einen Wandhalter für einen erfindungsgemässen Parfumdispenser;
- Figur 2b: eine Seitenansicht eines Wandhalters gemäss Fig. 2a für einen Parfumdispenser;
- Figur 3a: eine Ansicht von oben auf einen Parfumdispenser gemäss Figur 1b;
- Figur 3b: eine Ansicht von unten auf einen Parfumdispenser gemäss Fig 1b;
- Figur 4: eine Explosionsansicht der Bestandteile des Dispensers und einer Spraydose von vorne, wobei die Bestandteile teilweise geschnitten dargestellt sind; und
- Figur 5: eine Seitenansicht auf einen längsgeschnittenen Dispenser mit eingesetzter Spraydose und Wandhalter.

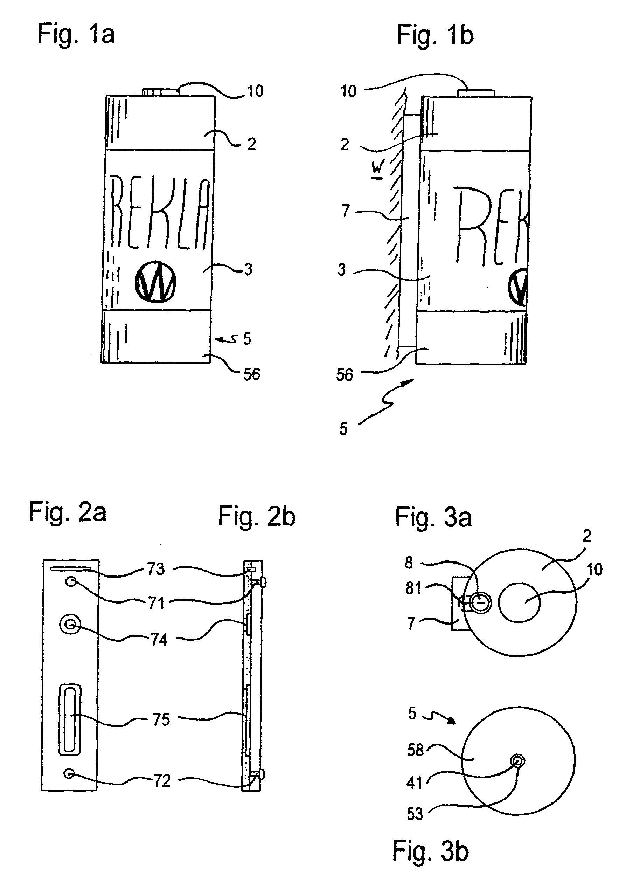

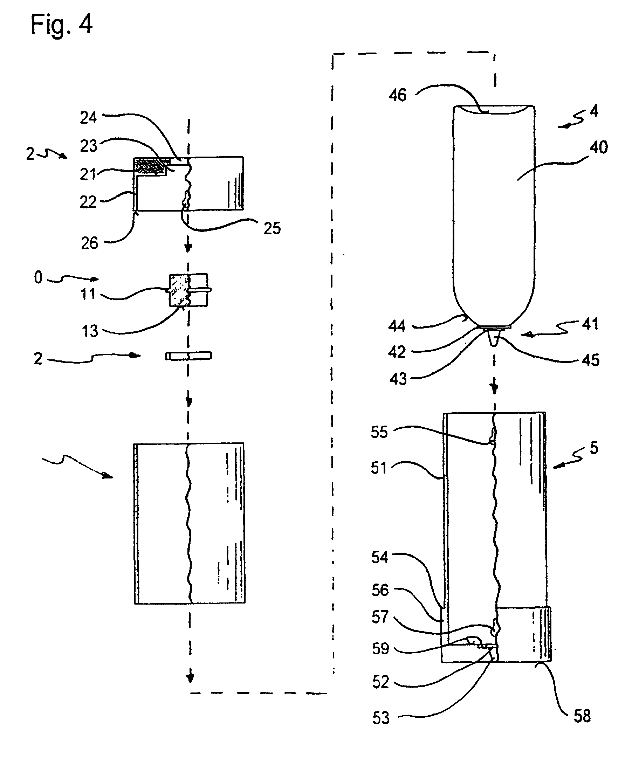

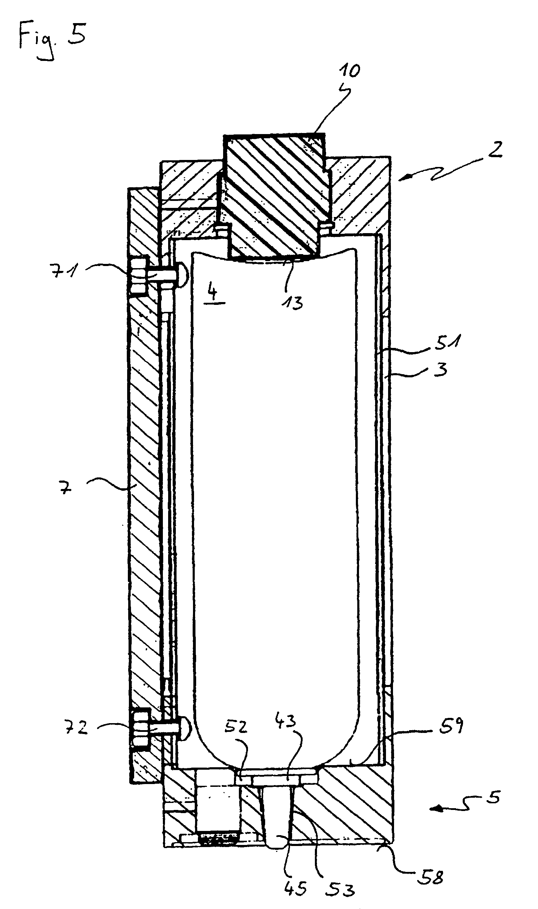

In Figur 1 ist ein Parfumdispenser skizziert wie er sich dem Benutzer von Vorne präsentiert. Ein Grundkörper 56 eines Bodens 5, ein Sichtmantel 3 und ein Deckel 2 bilden einen im wesentlichen zylinderförmigen Körper. Im Deckel 2 ist ein Knopf 10 gelagert, der vertikal beweglich den Deckel 2 durchsetzt. Ein oberer Bereich des Knopfes 10 ragt über die Deckeloberfläche nach oben und kann dadurch vom Benutzer bequem gedrückt werden. Durch seine prominente Position ist der Knopf 10 gut sichtbar und lädt geradezu zum Drücken ein. Dieser Effekt kann durch eine entsprechende kontrastierende Farbgebung noch verstärkt werden. Zwischen Boden 5 und Deckel 2 ist der transparente Mantel 3 angeordnet. Hinter dem Sichtmantel 3 können austauschbare Werbedrucke eingelegt sein. In der Seitenansicht nach Figur 1b ist gezeigt, wie der erfindungsgemässe Parfumdispenser 1 mittels einer Wandhalterung 7 lösbar an einer Wand W befestigt ist. Boden 5, Deckel 2 und Wandhalter 7 sind vorteilhafterweise aus Metall gefertigt. Wie aus den Figuren 4 und 5 ersichtlich ist, sitzt im zusammengebauten Zustand der Deckel 2 direkt auf einem oberen Bereich einer hochgezogenen Bodenwand 51 auf. Boden 5 und Deckel 2 bilden also ein stabiles hohlzylindrisches Metallgehäuse 1. Der Sichtmantel 3 besteht beispielsweise aus einem Acryl- oder Plexiglasrohr. Er ist über den Wandzylinder 51 gestülpt und liegt mit seiner Unterkante auf einem Absatz 54 auf. Der Absatz 54 wird durch den Übergang vom Wandzylinder 51 in den Bodengrundkörper 56 definiert. Der Wandzylinder 51 kann einstückig mit dem Bodengrundkörper 56 ausgebildet sein, oder er ist als separates Teil gefertigt und im Bodengrundkörper 56 befestigt. Im einfachsten Fall ist der Wandzylinder 51 ein passender Abschnitt eines Metallrohres, das in den Bodengrundkörper 56 eingeschweisst, eingeklebt oder eingepresst ist.

Der Innendurchmesser des Sichtmantels 3 und der Aussendurchmesser des Wandzylinders 51 sind so aufeinander abgestimmt, dass der Mantel 3 ohne übermässiges Spiel den Zylinder 51 umschliesst, aber gleichzeitig ein Blatt mit einem Werbeaufdruck zwischen beiden Platz findet. Die Höhe des transparenten Mantels 3 ist so bemessen, dass er im zusammengebauten Zustand genau zwischen die Anlagefläche 54 des Bodens 5 und eine Unterkante 26 einer herabgezogenen Seitenwand 22 des Deckels 2 passt. Die Aussendurchmesser von Bodengrundkörper 56, Mantel 3 und Deckel 2 sind annähernd gleich. Der Dispenser 1 bekommt dadurch nicht nur ein glattes schnörkelloses Aussehen, sondern das Fehlen von unnötigen Angriffsflächen trägt dazu bei Verschmutzungen und Beschädigungen zu vermeiden.
Der Deckel 2 ist mit einer zentralen Oeffnung 24 versehen, die von Knopf 10 durchsetzt ist. Die Oeffnung 24 ist in einem unteren Bereich 23 erweitert, in dem eine umlaufende Rippe 11 des Knopfes 10 zu liegen kommt. Einen Sicherungsring 12 hält die Rippe 11, und damit den Knopf 10, in der Oeffnung 24, 23 des Deckels 2, wobei der Knopf 10 in Vertikalrichtung mindestens einige Millimeter Spiel hat und auf- und abbewegt werden kann.
Der Zusammenbau des Dispensers 1 ist denkbar einfach. Eine Spraydose 4 wird in den Boden 5 eingeführt, so dass ein Spraykopf 41 in einer zentralen Oeffnung 53 des Bodens 5 zu liegen kommt. Die Spraydose 4 steckt also mit der Abgabeöffung nach unten im Dispenser 1. Der Mantel 3 wird mit innenliegendem Werbeblatt über den Boden gestülpt und anschliessend wird der Deckel 2 mit dem bereits eingebauten Knopf 10 aufgesetzt. Im zusammengebauten Zustand kommt eine Einhängeöffnung 25 des Deckels 2 deckungsgleich auf einer gleichförmigen Einhangeöffnung 55 der Wand 51 zu liegen.
Die Spraydose 4 umfasst einen zylindrischen Druckbehälter 40 vorzugsweise aus innenbeschichtetem Metallblech und einen Spraykopf 41. Wie bereits erwähnt sind Spraydosen, wie sie in der vorliegenden Erfindung zum Einsatz kommen, so modifiziert, dass beim Einsatz ihr Sprühkopf 41 nach unten weist und das Aerosol nach unten abgegeben wird. Dazu ist unter anderem das Steigrohr im inneren der Dosen stark verkürzt und das Sprühventil modifiziert. Der Spraykopf 41 umfasst einen Flansch 43 und einen Konus 45 die von einer zentralen Oeffnung durchsetzt sind, durch die das Aerosol bei Bedarf abgegeben wird.
Wie in der Figur 5 dargestellt ist, sind die Grösse der Spraydose 4 und die Gehäusegrösse so aufeinander abgestimmt, dass ein unterer Bereich 13 des Knopfes 10 auf oder knapp über einer oberen Wand 46 des Druckbehälters 40 der Dose 4 zu liegen kommt. Das entgegengesetzte Ende der Dose 4 ist mit dem Konus 45 des Spraykopfes 41 in die Abgabeöffnung 53 eingeführt. Diese Oeffnung 53 des Bodens 5 verjüngt sich nach unten hin und weist in ihrem oberen Bereich eine stufenartigen Erweiterung 52 auf. In der Erweiterung 52 kommt der scheibenförmige Flansch 43 des Spraykopfes 41 zu liegen. Durch Druck auf den Knopf 10 wird der Druckbehälter 40 der Spraydose 4 nach unten gegen den Spraykopf 41 verschoben. Die Grundfläche der Erweiterung 52 im Boden 5 dient dabei als Widerlager für den Spraykopf 41. Durch die Relativbewegung des Druckbehälters 40 zum Spraykopf 41 wird ein, in den Zeichnungen nicht dargestelltes, Ventil geöffnet und Aerosol fein zerstäubt durch den Konus 45 nach unten abgegeben. Der Druckbehälter 40 kann nur einige Millimeter nach unten gedrückt werden bevor Schultern 44 des Druckbehälters 40 an der Grundfläche 59 des Bodens 5 anschlagen. In anderen vorteilhaften Ausführungen kann der Weg den der Druckbehälter 40 maximal zurücklegen kann, durch das Zusammenwirken eines Wulstes 42 des Druckbehälters 40 mit Grundfläche 59 des Bodens 5 oder indirekt durch ein Zusammenwirken mit dem Flansch 43 des Spraykopfes 41 begrenzt werden.
Beim Nachlassen oder Wegfallen des Druckes auf den Knopf 10 genügt der Innendruck von ca. 8 bar im Druckbehälter 40 um das Ventil zu schlissen und Druckbehälter 40 und Knopf 10 wieder in ihre obere Ruheposition zu heben.
Das Dispensergehäuse 1 weist also mit Knopf 10 nur ein einziges bewegliches Bauteil auf.
In einer vorteilhaften Ausführungsform der Erfindung, wie sie in der Figur 5 dargestellt ist, gibt praktisch kein bewegliches Bauteil mehr. Der Knopf 10 ist aus elastischem Kunststoffmaterial gefertigt und nicht mehr im eigentlichen Sinne hin- und herbeweglich in der Oeffnung 23, 24 im Deckel 2 gehalten. Er füllt diese vielmehr vollständig aus und wird durch Druck auf die Knopfoberseite elastisch verformt. Die Verformung genügt um die Knopfunterseite 13 mit genügend Druck auf den Druckbehälter 40 zu pressen und den Sprayvorgang auszulösen. Bei einem solchen elastischen Knopf 10 kann es zu keinem Verklemmen mehr kommen. Ausserdem ist der Deckel 2 dicht gegen den Knopf 10 abgeschlossen und von oben können keine Verunreinigungen in das Gehäuse eindringen.

Tests haben gezeigt, dass eine Spraydose mit einem Volumen von 500 ml etwa 5000 Anwendungen zulässt. Die Zeitintervalle, in denen die Spraydosen ersetzt werden müssen, können daher auch in stark frequentierten Örtlichkeiten sehr lange gehalten werden.

Ist der Dispenser 1 mit Spraydose und Werbeblatt bestückt und zusammengebaut, so kann er in die Wandhalterung 7 eingehängt werden. Dazu dienen in bekannter Weise ein oberer 71 und ein unterer 72 Sicherungsstift mit erweitertem Kopf, welche in die oberen 55, 25 und die untere 57 Einhängeöffnung eingehängt werden. Um zu verhindern, dass der Dispenser von unbefugten Personen ausgehängt wird, ist im Deckel 2 ein Schloss 8 angebracht, dessen Riegel 81 in eine Nut 73 am Wandhalter 7 eingreifen kann, so dass eine Vertikalbewegung des Dispensers nicht mehr möglich ist. Wie ebenfalls in der Figur 2 dargestellt ist, sind zur Befestigung des Wandhalters 7 an einer Wand geeignete Bohrungen 74 und Langlöcher 75 vorgesehen.
Stall an einer Wand kann der Dispenser natürlich auch an einer Säule oder einem Ständer montiert sein.

Der erfindungsgemässe Dispenser ermöglicht es in idealer Weise beim Konsumenten die visuelle und olfaktorische Wahrnehmung des Produktes zu verknüpfen und damit den Werbeeffekt enorm zu steigern. Der Benutzer kann einerseits durch das Werbeblatt auf die Marke des Parfums aufmerksam gemacht und zum Gebrauch des Dispensers angeregt werden, andererseits hat er vor, während und nach dem Besprühen der Hände sowie beim anschliessenden Verteilen des Parfums auf gewünschte Körperpartien die Produktinformation voll im Blick.
Die stabile und äusserst einfache Bauweise des Dispensers 1 schützt Dispenser und Inhalt vor Vandalismus und Diebstahl so dass auch nach längerem Einsatz in stark frequentierten Lokalitäten die einwandfreie Funktion und das einwandfreie Aussehen gewährleistet sind. Die Materialkombination Plexiglas-Metall und die schlichte Formgebung gestatten es, trotz der hohen Anforderungen an Stabilität und Funktionalität, einen Dispenser zu schaffen dessen elegantes Aussehen die Werbewirkung für Hochpreisprodukt wie Parfum noch zusätzlich unterstützt.
Alle Oberflächen lassen sich sehr gut reinigen, so dass der Dispenser einfach sauberzuhalten ist und immer zum Gebrauch einlädt. Ventil und Spraykopf 41 der Spraydose 4 sind tropfarm ausgebildet. Tropfen von Parfumresten die dennoch entstehen verdunsten entweder vom Spraykopf respektive von der Unterseite des Dispensers, oder sie tropfen nach unten.

## Patentansprüche

1. Parfumdispenser zur dosierten Abgabe eines feinverteilten Aerosols, welcher einen Boden (5), ein Deckel (2) und eine doppelwandige Seitenwandung (51, 3) umfasst, wobei Boden (5), Deckel (2) und eine innere Seitenwand (51) ein stabiles verschliessbares Gehäuse (1) zur Aufnahme einer Spraydose (4) bilden, welche von dem Gehäuse (1) vollständig umgeben wird, **dadurch gekennzeichnet, dass** die innere Seitenwand (51) in einem von aussen sichtbaren Bereich zwischen Boden (5) und Deckel (2) von einem transparenten Sichtmantel (3) umgeben ist, und in der doppelwandigen Seitenwandung zwischen Sichtmantel (3) und innerer Seitenwand (51) ein Werbeblatt anbringbar ist, und dass der Deckel (2) eine zentrale Oeffnung (23, 24) aufweist welche von einem Knopf (10) durchsetzt ist und dass der Boden (5) eine zentrale Oeffnung (53) aufweist, in die ein Spraykopf (41) einer Spraydose (4) einführbar ist.

2. Parfumdispenser nach Anspruch 1, **dadurch gekennzeichnet, dass** die Seitenflächen von Boden (5), Deckel (2) und Mantel (3) eine kreiszylindrische Mantelfläche definieren.

3. Parfumdispenser nach Anspruch 1, **dadurch gekennzeichnet, dass**, das zylindrische Gehäuse aus Boden (5), Deckel (2) und Doppelwand (51, 3) an einer Halterung (7) lösbar befestigbar ist.

4. Parfumdispenser nach einem der Ansprüche 1 bis 4, **dadurch gekennzeichnet, dass** der Deckel (2) lösbar auf der inneren Seitenwand (51) befestigbar ist, wobei ein oberer Bereich der inneren Seitenwand (51) von einer herabgezogenen Seitenwand (22) des Deckels (2) umschlossen ist und ein unterer Bereich der inneren Seitenwand (51) von einer hochgezogenen Seitenwand (56) des Bodens (5) umschlossen und daran befestigbar ist.

5. Parfumdispenser nach Anspruch 4, **dadurch gekennzeichnet, dass**, im zusammengebauten Zustand eine Einhängeöffnung (25) in der Seitenwand (22) des Deckels (2) mit einer gleichgestalteten Einhängeöffnung (55) in der inneren Seitenwand (51) zur Deckung bringbar ist, und eine so gebildete obere Einhängeöffnung (25, 55) und eine untere Einhängeöffnung (57) mit Sicherungsstiften (71, 72) der Halterung (7) in Wirkverbindung bringbar sind.

6. Parfumdispenser nach Anspruch 4, **dadurch gekennzeichnet, dass** im Deckel (2) ein Schloss (8) angebracht ist, wobei in geschlossenem Zustand ein Riegel (81) des Schlosses (8) in eine Nut (73) der Halterung (7) eingreift und das zylindrische Gehäuse (1) mit der Halterung (7) verriegelt.

7. Parfumdispenser nach Anspruch 1, **dadurch gekennzeichnet, dass** der Knopf (10) oben und unten aus dem Deckel (2) hervorragt und entweder in der zentralen Oeffnung (23, 24) des Deckels (2) auf- und ab beweglich gelagert ist, oder dass der Knopf (10) den Deckel (2) durchsetzt und die zentrale Oeffnung (23, 24) annähernd vollständig ausfüllt und aus einem Elastomermaterial gefertigt ist.

8. Parfumdispenser nach Anspruch 2, **dadurch gekennzeichnet, dass** Boden (5), Deckel (2) und innere Seitenwand (51) aus massiven Metallteilen gefertigt sind und der Sichtmantel aus Plexi- oder Acrylglas besteht.

9. Parfumdispenser nach Anspruch 1 mit einer eingebauten Spraydose (4), wobei die Spraydose (4) einen Druckbehälter (40) mit einer Behälterwand (46) und einem Spraykopf aufweist (41), der Spraykopf (41) einen Flansch aufweist und die Öffnung (53) im Gehäuseboden (5) eine obere stufenartige Erweiterung (52) aufweist, wobei die Spraydose (4) im Gehäuse (1) angeordnet und von diesem schützend umschlossen ist, so dass die obere Behälterwand (46) des Druckbehälters (40) der Spraydose (4) direkt unter der Unterseite (13) des im Deckel (2) angeordneten Knopfes (10) zu liegen kommt und der Spraykopf (41) die Öffnung (53) im Boden (5) durchsetzt, und die obere stufenartige Erweiterung (52) der Öffnung (53) den Flansch (43) des Spraykopfes (41) aufnimmt und als Widerlager dient.

## Claims

1. A perfume dispenser for the metered dispensing of a finely dispersed aerosol which comprises a base (5), a lid (2) and a double-walled side walling (51,3), wherein base (5), lid (2) and an inner side wall (51) form a stable, closable housing (1) for accommodating a spray can (4) which is completely surrounded by the housing (1), **characterised in that** the inner side wall (51) in a region which is between the base (5) and the lid (2) and is visible from the outside is surrounded by a transparent viewing casing (3), and in the double-walled side walling between the viewing casing (3) and the inner side wall (51) there may be attached an advertisement print, and that the lid (2) comprises a central opening (23, 24) which is passed through by a button (10) and that the base (5) comprises a central opening (53) into which a spray head (41) of a spray can (4) may be introduced.

2. A perfume dispenser according to claim 1, **characterised in that** the side surfaces of the base (5), lid (2) and casing (3) define a circular-cylindrical casing surface.

3. A perfume dispenser according to claim 1, **characterised in that** the cylindrical housing of the base (5), lid (2) and double wall (51, 3) is releasably fastenable on a mounting (7).

4. A perfume dispenser according to one of the claims 1 to 4, **characterised in that** the lid (2) is releasably fastenable on the inner side wall (51), wherein an upper region of the inner side wall (51) is enclosed by a downwardly projecting side wall (22) of the lid (2) and a lower region of the inner side wall (51) is enclosed by an upwardly projecting side wall (56) of the base (5) and may be fastened thereon.

5. A perfume dispenser according to claim 4, **characterised in that** in the constructed condition, a hanger opening (25) in the side wall (22) of the lid (2) may be brought to overlap with an equally designed hanger opening (55) in the inner side wall (51), and a thus formed upper hanger opening (25, 55) and lower hanger opening (57) may be brought into interactive connection with the securing pins (71, 72) of the mounting (7).

6. A perfume dispenser according to claim 4, **characterised in that** in the lid (2) there is arranged a lock (8), wherein in the closed condition a bar (81) of the lock (8) engages into a groove (73) of the mounting (7), and the cylindrical housing (1) locks with the mounting (7).

7. A perfume dispenser according to claim 1, **characterised in that** the button (10) above and below projects out of the lid (2) and either is mounted moveable up and down in the central opening (23, 24) of the lid (2), or that the button (10) passes through the lid (2) and fills the central opening (23, 24) approximately completely and is manufactured of an elastomer material.

8. A perfume dispenser according to claim 2, **characterised in that** the base (5), lid (2) and the inner side wall (51) consist of solid metal parts and the viewing casing consists of plexi-glass or acrylic glass.

9. A perfume dispenser according to claim 1, with an integrated spray can (4), wherein the spray can (4) comprises a pressure container (40) with a container wall (46) and with a spray head (41), the spray head (41) comprises a flange and the opening (53) in the housing base (5) comprises an upper step-like extension (52), wherein the spray can (4) is arranged in the housing (1) and is enclosed by this in a protective manner so that the upper container wall (46) of the pressure container (40) of the spray can (4) comes to lie directly below the underside (13) of the button (10) arranged in the lid (3) and the spray head (41) passes through the opening (53) in the base (5), and the upper step-like extension (52) of the opening (53) accommodates the flange (43) of the spray head (41) and serves as a counter bearing.

## Revendications

1. Diffuseur de parfum pour l'émission dosée d'un aérosol finement dispersé, lequel diffuseur comprend un fond (5), un couvercle (2), et une paroi latérale à double paroi (51, 3), le fond (5), le couvercle (2) et une paroi latérale (51) interne formant un boîtier (1) stable pouvant être obturé pour recevoir une bombe aérosol (4), laquelle est entièrement entourée par le boîtier (1), **caractérisé en ce que** la paroi latérale (51) interne est entourée par une jaquette de visibilité (3) transparente dans une partie visible de l'extérieur entre le fond (5) et le couvercle (2), et qu'un prospectus publicitaire peut être introduit dans la paroi latérale à double paroi entre la jaquette de visibilité (3) et la paroi latérale (51) interne, **et en ce que le** couvercle (2) présente une ouverture (23, 24) centrale, laquelle est traversée par un bouton (10) **et en ce que** le fond (5) présente une ouverture centrale (53) dans laquelle une tête de vaporisation (41) d'une bombe aérosol (4) peut être introduite.

2. Diffuseur de parfum selon la revendication 1, **caractérisé en ce que** les surfaces latérales du fond (5), du couvercle (2) et de la jaquette (3) définissent une surface de jaquette cylindrique circulaire.

3. Diffuseur de parfum selon la revendication 1, **caractérisé en ce que** le boîtier cylindrique constitué par le fond (5), le couvercle (2) et la paroi double (51, 3) peut être fixé à une fixation (7) de manière amovible.

4. Diffuseur de parfum selon l'une des revendications 1 à 4, **caractérisé en ce que** le couvercle (2) peut être fixé de manière amovible sur la paroi latérale (51) interne, une partie supérieure de la paroi latérale (51) interne étant entourée par une paroi latérale (22) du couvercle (2) abaissée et une partie inférieure de la paroi latérale (51) interne étant entourée par une paroi latérale (56) du fond (5) relevée et pouvant y être fixée.

5. Diffuseur de parfum selon la revendication 4, **caractérisé en ce que,** à l'état monté, une ouverture d'accrochage (25) dans la paroi latérale (22) du couvercle (2) peut coïncider avec une ouverture d'accrochage (55) réalisée de la même manière dans la paroi latérale (51) interne, et qu'une ouverture d'accrochage (22, 55) supérieure ainsi formée et une ouverture d'accrochage (57) inférieure peuvent être raccordées de manière effective avec des goupilles à ressort (71, 72) de la fixation (7).

6. Diffuseur de parfum selon la revendication 4, **caractérisé en ce qu'une** serrure (8) est montée dans le couvercle (2), un verrou (81) de la serrure (8) ayant prise, à l'état fermé, dans une rainure (73) de la fixation (7) et le boîtier (1) cylindrique se verrouillant alors avec la fixation (7).

7. Diffuseur de parfum selon la revendication 1, **caractérisé en ce que** le bouton (10) dépasse en haut et en bas hors du couvercle (2) et que soit il est placé de manière mobile vers le haut et vers le bas dans l'ouverture centrale (23, 24) du couvercle (2), soit le bouton (10) traverse le couvercle (2) et remplit presque entièrement l'ouverture centrale (23, 24) et est fabriqué dans un matériau élastomère.

8. Diffuseur de parfum selon la revendication 2, **caractérisé en ce que** le fond (5), le couvercle (2) et la paroi latérale (51) interne sont fabriqués de pièces métalliques massives et que la jaquette de visibilité est constituée de Plexiglas ou de verre acrylique.

9. Diffuseur de parfum selon la revendication 1, avec une bombe aérosol (4) intégrée, la bombe aérosol (4) présentant un réservoir sous pression (40) avec une paroi de réservoir (46) et une tête de vaporisateur (41), la tête de vaporisateur (41) présentant une bride, et l'ouverture (53) présentant un élargissement (52) supérieur en forme d'échelon dans le fond du boîtier (5), la bombe aérosol (4) étant placée dans le boîtier (1) et étant entourée de manière protectrice par celui-ci, de sorte que la paroi supérieure (46) du réservoir sous pression (40) de la bombe aérosol (4) vient se placer directement sous le côté inférieur (13) du bouton (10) placé dans le couvercle (2) et que la tête de vaporisation (41) traverse l'ouverture (53) dans le fond (5), et que l'élargissement (52) supérieur en forme d'échelon de l'ouverture (53) loge la bride (43) de la tête de vaporisation (41) et sert de contre-butée.
